# FASCICULE DE BREVET EUROPEEN

(11) **EP 2 225 245 B1**
(45) Date de publication et mention de la délivrance du brevet: **19.10.2011**
(21) Numéro de dépôt: 08873376.1
(22) Date de dépôt: 31.12.2008
(51) Int. Cl.: C07D 471/04, A61K 31/437, A61P 25/00, A61P 35/00, A61P 19/10, A61P 29/00, C07D 213/74, C07D 213/70, C07D 213/71, C07D 233/64

(54) **Composés de N-phényl-imidazo(1,2-a)pyridine-2-carboxamides, leur préparation et leur application en thérapeutique**
N-Phenyl-Imidazo(1,2-a)pyridincarboxamid-Verbindungen, ihre Herstellung sowie ihre therapeutische Anwendung
N-phenyl-imidazo(1,2-a)pyridine-2-carboxamide compounds, preparation thereof and therapeutic application thereof

(30) Priorité: 02.01.2008 FR 0800008
(43) Date de publication de la demande: 08.09.2010
(73) Titulaire: SANOFI, 75013 Paris (FR)
(72) Inventeur: PEYRONEL, Jean-François, F-75013 Paris (FR)
(74) Mandataire: Le Coupanec, Pascale A.M.P.
(86) Numéro de dépôt international: PCT/FR2008/001839
(87) Numéro de publication internationale: WO 2009/115651

(56) Documents cités:
- WO-A-2005/030705
- WO-A-2008/003856
- FR-A- 2 638 161
- US-A1- 2005 165 049
- US-A1- 2005 239 800
- FISHER M H ET AL: "IMIDAZO(1,2-a)PYRIDINE ANTHELMINTIC AND ANTIFUNGAL AGENTS" JOURNAL OF MEDICINAL CHEMISTRY, AMERICAN CHEMICAL SOCIETY, WASHINGTON, US, vol. 15, no. 9, 1 janvier 1972 (1972-01-01), pages 982-985, XP001105353 ISSN: 0022-2623

## Description

La présente invention se rapporte à des dérivés d'imidazo[1,2-*a*]pyridine-2-carboxamides, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

La présente invention a pour objet les composés de formule (I) : 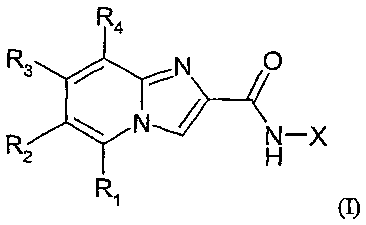 dans laquelle :
X représente un groupe phényle éventuellement substitué par un ou plusieurs groupes choisis indépendamment les uns des autres parmi les atomes ou groupes suivants : halogène, (C₁-C₆)alcoxy, (C₁-C₆)alkyle, NRaRb, les groupes alkyle et alcoxy pouvant éventuellement être substitués par un ou plusieurs atomes d'halogène ;
R₁ représente un atome d'hydrogène, un atome d'halogène, un groupe (C₁-C₆)alcoxy, un groupe (C₁-C₆)alkyle, un amino, un groupe NRcRd; les groupes alkyle et alcoxy pouvant éventuellement être substitués par un ou plusieurs atomes d'halogènes, un groupe hydroxy, amino, ou groupe (C₁-C₆)alcoxy ;
R₂ représente l'un des groupes suivants :
   - un groupe NRcRd,
   - un groupe -N=CH-NRaRb,
   - nitro, hydroxyiminoalkyle, alcoxyiminoalkyle,
   - un groupe (C₁-C₆)alkylthio,
   - un groupe (C₁-C₆)alkylsulfinyle,
   - un groupe (C₁-C₆)alkylsulfonyle,
   - un groupe -SO₂-NR₅R₆,
   - un groupe (((C₁-C₆)alkyl)₃)silyléthynyle ;
R₃ représente un atome d'hydrogène, un groupe (C₁-C₆)alkyle, un groupe (C₁-C₆)alcoxy ou un atome d'halogène,
R₄ représente un atome d'hydrogène, un groupe (C₁-C₄)alkyle, un groupe (C₁-C₄)alcoxy ou un atome de fluor ;
R₅ et R₆, identiques ou différents, représentent un atome d'hydrogène ou un groupe (C₁-C₆)alkyle,
Ra et Rb représentent, indépendamment l'un de l'autre, un atome d'hydrogène, un groupe (C₁-C₆)alkyle ou forment avec l'atome d'azote qui les porte un cycle de 4 à 7 chaînons,
Rc représente un atome d'hydrogène et Rd représente un atome d'hydrogène ou un groupe (C₁-C₆)alkyle,
à l'état de base ou de sel d'addition à un acide.

On connaît du document FR 2 638 161 les composés dérivés de benzoyl-2-imidazo[1,2-a]pyridine, utiles en tant que médicaments.

On connaît également du document US 2005/0239800 l'utilisation de pyrazines polycycliques comme modulateurs de potassium des canaux ioniques.

WO 2005/030705 décrit des ortho-amino benzamides inhibiteurs d'histone deacetylase.

WO 2008/003856 décrit la préparation et l'utilisation de dérivés d'imidazo [1,2-*a*]pyridine-2-carboxamides.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans la cadre de la présente invention, on entend par :
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl etc ;
- un groupe alcényle : un groupe aliphatique mono- ou poly-insaturé, linéaire ou ramifié, comprenant par exemple une ou deux insaturations éthyléniques ;

- un groupe alcoxy: un radical -O-alkyle où le groupe alkyle est tel que précédemment défini ;
- un groupe alcynyle : un groupe aliphatique mono- ou poly-insaturé, linéaire ou ramifié, comprenant par exemple une ou deux insaturations éthylyniques.

Différents sous-ensembles de composés sont définis ci-après, faisant également partie de l'invention.

Parmi les composés de formule générale (I) objets de l'invention telle que définie précédemment, un premier groupe de composés est constitué par les composés pour lesquels au moins l'un des groupements R₁ ou R₂ est différent d'un atome d'hydrogène ; les autres groupes étant tels que définis précédemment.

Parmi les composés de formule générale (I) objets de l'invention telle que définie précédemment, un deuxième groupe de composés est constitué par les composés pour lesquels R₁ représente un atome d'hydrogène, un atome d'halogène, un groupe (C₁-C₆)alkyle, un groupe (C₁-C₆)alcoxy ; les autres groupes étant tels que définis précédemment.

Parmi les composés de formule générale (I) objets de l'invention telle que définie précédemment, un troisième groupe de composés est constitué par les composés pour lesquels X représente un groupe phényle ; les autres groupes étant tels que définis précédemment.

Parmi les composés de formule générale (I) objets de l'invention telle que définie précédemment, un quatrième groupe de composés est constitué par les composés pour lesquels R₃ et R₄ représentent chacun un atome d'hydrogène ; les autres groupes étant tels que définis précédemment.

Parmi les composés de formule générale (I) objets de l'invention telle que définie précédemment, un cinquième groupe de composés est constitué par les composés pour lesquels R₂ représente l'un des groupes suivants :
- un groupe NRcRd,
- un groupe -N=CH-NRaRb,
- nitro, hydroxyiminoalkyle, alcoxyiminoalkyle,
- un groupe (C₁-C₆)alkylthio,
- un groupe (C₁-C₆)alkylsulfinyle,
- un groupe (C₁-C₆)alkylsulfonyle,
- un groupe -SO₂-NR₅R₆,
- un groupe (((C₁-C₆)alkyl)₃)silyléthynyle ;
R₅ et R₆, identiques ou différents, représentent un atome d'hydrogène ou un groupe (C₁-C₆)alkyle ;
Ra et Rb représentent, indépendamment l'un de l'autre, un atome d'hydrogène ou un groupe (C₁-C₆)alkyle ;
Rc représente un atome d'hydrogène et Rd représente un atome d'hydrogène ou un groupe (C₁-C₆)alkyle, ledit alkyle étant éventuellement substitué par un groupe (C₁-C₆) alcoxy ;
les autres groupes étant définis tels que précédemment.

Parmi les composés de formule générale (I) objets de l'invention telle que définie précédemment, un sixième groupe de composés est constitué par les composés pour lesquels R₂ représente l'un des groupes suivants :
NH₂, CH=NOH, NHiPr, nitro, CH=NOMe, NHMe, N=CHNMe₂, NHEt, NHCH₂CH₂OMe, SMe, SOMe, SO₂Me, SO₂NH₂, SO₂NHMe, SO₂NMe₂, C≡CSiMe₃ ;
et les autres groupes étant définis tels que précédemment.

Parmi les composés de formule (I) objets de l'invention, un septième groupe de composés est constitué des composés pour lesquels :
X représente un groupe phényle ;
R₁ représente un atome d'hydrogène, un atome de chlore, un groupe méthyle ou éthoxy ; R₃ et R₄ représentent un atome d'hydrogéne ;
R₂ représente un groupe NH₂, CH=NOH, NHiPr, nitro, CH=NOMe, NHMe, N=CHNMe₂, NHEt, NHCH₂CH₂OMe, SMe, SOMe, SO₂Me, SO₂NH₂, SO₂NHMe, SO₂NMe₂, C≡CSiMe₃ ;
à l'état de base ou de sel d'addition à un acide.

Parmi les composés de formule (I) objets de l'invention, on peut notamment citer les composés suivants :
- 6-[(E)-(hydroxyimino)méthyl]-*N*-phénylimidazo[1,2-*a*]pyridine-2-carboxamide
- 6-(Isopropylamino)-*N*-phénylimidazo[1,2-*a*]pyridine-2-carboxamide et son chlorhydrate (1:1)
- 6-Nitro-*N*-phénylimidazo[1,2-*a*]pyridine-2-carboxamide
- 6-Amino-*N*-phénylimidazo[1,2-*a*]pyridine-2-carboxamide
- 6-Amino-5-chloro-*N*-phénylimidazo[1,2-*a*]pyridine-2-carboxamide
- 6-Amino-5-éthoxy-*N*-phénylimidazo[1,2-*a*]pyridine-2-carboxamide
- 6-{[(1E)-(diméthylamino)méthylène]amino}-*N*-phénylimidazo[1,2-*a*]pyridine-2-carboxamide
- 6-[(E)-(méthoxyimino)méthyl]-*N*-phénylimidazo[1,2-*a*]pyridine-2-carboxamide
- 6-(Méthylamino)-*N*-phénylimidazo[1,2-*a*]pyridine-2-carboxamide
- 6-(Ethylamino)-*N*-phénylimidazo[1,2-*a*]pyridine-2-carboxamide
- 6-(2-Méthoxyéthylamino)-*N*-phénylimidazo[1,2-*a*]pyridine-2-carboxamide
- 5-Méthyl-6-nitro-*N*-phénylimidazo[1,2-*a*]pyridine-2-carboxamide
- 6-Amino-5-méthyl-*N*-phénylimidazo[1,2-*a*]pyridine-2-carboxamide
- 6-(Méthylthio)-*N*-phénylimidazo[1,2-*a*]pyridine-2-carboxamide et son bromhydrate (1:1)
- 6-[(RS)-méthylsulfinyl]-*N*-phénylimidazo[1,2-*a*]pyridine-2-carboxamide et son bromhydrate (1:1)
- 6-(Méthylsulfonyl)-*N*-phénylimidazo[1,2-*a*]pyridine-2-carboxamide et son bromhydrate (1:1)
- 6-(Aminosulfonyl)-*N*-phénylimidazo[1,2-*a*]pyridine-2-carboxamide et son bromhydrate (1:1)
- 6-[(Méthylamino)sulfonyl]-*N*-phénylimidazo[1,2-*a*]pyridine-2-carboxamide et son bromhydrate (1:1)
- 6-[(Diméthylamino)sulfonyl]-*N*-phénylimidazo[1,2-*a*]pyridine-2-carboxamide
- 6-(Triméthylsilyléthynyl)-*N*-phénylimidazo[1,2-*a*]pyridine-2-carboxamide et son bromhydrate (1:1)

Les sels d'addition à un acide de ces composés font également partie de la présente invention.

Conformément à l'invention, on peut préparer les composés de formule générale (I) selon le procédé décrit dans le schéma 1.

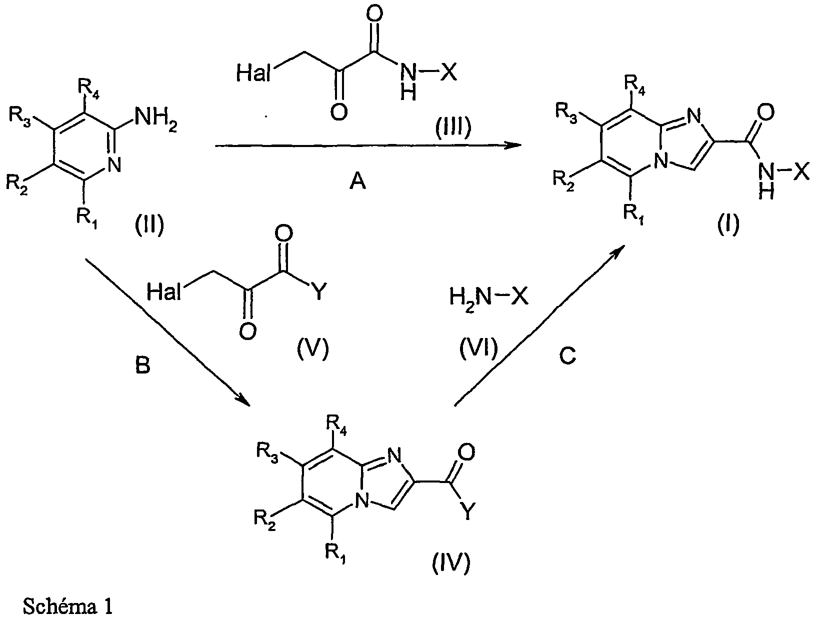

La voie A consiste à préparer les 2-amino-pyridines de formule (II) selon les méthodes connues de l'homme du métier et à former le cycle imidazo [1,2-*a*]pyridine par condensation sur un dérivé de 2-oxo-*N*-aryl-propionamide (III) dans lequel Hal représente un atome de chlore, de brome ou d'iode et X est défini comme précédemment, par analogie avec les méthodes décrites par J-J. Bourguignon et coll. dans Aust. J. Chem., 50, 719 (1997) et par J.G. Lombardino dans J. Org. Chem., 30, 2403 (1965) par exemple. Les dérivés halogénés de 2-oxo-*N*-aryl-propionamide (III) peuvent être obtenus selon la méthode décrite par R. Kluger et coll. dans J. Am. Chem. Soc., 106, 4017 (1984).

La seconde voie de synthèse B, C consiste à coupler un acide imidazopyridine-2-carboxylique ou l'un de ses dérivés, de formule (IV), dans laquelle Y est hydroxy, halogène ou (C₁-C₆)alcoxy avec une arylamine X-NH₂ de formule (VI), dans laquelle X est défini comme précédemment, selon des méthodes connues de l'homme du métier. Ainsi l'acide peut être préalablement converti en l'un de ses dérivés réactifs tel que : halogénure d'acide, anhydride, anhydride mixte ou ester activé puis mis en réaction avec l'amine (VI), en présence d'une base telle que la diisopropyléthylamine, la triéthylamine ou la pyridine, dans un solvant inerte tel que le THF, le DMF ou le dichlorométhane. Le couplage peut également être réalisé en présence d'un agent de couplage tel que CDI, EDCI, HATU ou HBTU dans les mêmes conditions sans isoler d'intermédiaire réactif. Alternativement on peut faire réagir l'amine (VI) avec un ester de l'acide de formule (IV) en présence d'un catalyseur tel que le triméthylaluminium selon la méthode de Weinreb, S. et coll (Tet. Lett., 18, 4171 (1977)) ou le terbutylate de zirconium.

Les acides imidazopyridine-2-carboxyliques et leurs dérivés de formule (IV) peuvent être obtenus en condensant les 2-aminopyridines appropriées sur un ester de l'acide 3-halogèno-2-oxo-propionique selon la méthode décrite par J.G. Lombardino dans J. Org. Chem., 30(7), 2403 (1965), puis en déprotégeant l'ester en acide et convertissant le cas échéant l'acide en l'un de ses dérivés.

Les produits de formule (I) et leurs précurseurs de formule (II) ou (IV), peuvent être soumis, si désiré et si nécessaire, pour obtenir des produits de formule (I) ou être transformés en d'autres produits de formule (I) à l'une ou plusieurs des réactions de transformations suivantes, dans un ordre quelconque :
a) une réaction d'estérification ou d'amidification de fonction acide,
b) une réaction d'amidification de fonction amine,
c) une réaction d'hydrolyse de fonction ester en fonction acide,
d) une réaction de transformation de fonction hydroxyle en fonction alcoxy,
e) une réaction d'oxydation de fonction alcool en fonction aldéhyde ou cétone,
f) une réaction de conversion des fonctions aldéhyde ou cétone en dérivé oxime,
g) une réaction de transformation de radical nitrile en fonction aldéhyde,
h) une réaction de transformation de radical nitrile en fonction cétone,
i) une réaction d'oxydation de groupe alcènyle en fonction aldéhyde ou cétone,
j) une réaction de réduction d'un groupe nitro en groupe amino primaire,
k) une réaction de conversion d'un groupe amino primaire ou secondaire en un groupe amino, secondaire ou tertiaire par amination réductrice ou alkylation,
l) une réaction de conversion d'un groupe amino primaire en un groupe amidine,
m) une réaction d'oxydation de fonction alkylthioéther en fonction alkylsulfoxyde ou alkylsulfone,
n) une réaction d'oxydation de fonction alkylsulfoxyde en fonction alkylsulfone,
o) une réaction de protection des fonctions réactives,
p) une réaction d'élimination des groupements protecteurs que peuvent porter les fonctions réactives protégées,
q) une réaction de salification par un acide minéral ou organique ou par une base pour obtenir le sel correspondant,
r) une réaction de dédoublement des formes racémiques en énantiomères,
   lesdits produits de formule (I) ainsi obtenus étant le cas échéant sous toutes les formes isomères possibles racémiques, énantiomères et diastéréoisomères, tautomères.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. numéros des composés exemplifiés renvoient à ceux donnés dans le tableau ci-après, qui illustre les structures chimiques et les propriétés physiques de quelques composés selon l'invention.

### Exemple 1 : 6-[(E)-(hydroxyimino)méthyl]-N-phénylimidazo[1,2-a]pyridine-2-carboxamide

Une solution de 123 mg de 6-formyl-*N*-phénylimidazo[1,2-*a*]pyridino-2-carboxamide et 48 mg de chlorhydrate d'hydroxylamine dans 6,5 mL de pyridine est agitée pendant 2 heures à 20°C puis concentrée à sec sous pression réduite. Le solide est lavé à l'eau puis à l'éther diéthylique et dissout dans un mélange de 75 mL de méthanol et 75 mL de dichlorométhane additionné de 2 mL de pyridine La solution est évaporée en présence de 1,5 g de silice. Après chromatographie sur une colonne de silice en éluant par un mélange de cyclohexane et d'acétate d'éthyle. Les fractions contenant le produit attendu sont réunies et évaporées à sec sous pression réduite. Le solide est trituré avec de l'acétate d'éthyle, filtré, lavé par de l'acétate d'éthyle puis de l'éther diéthylique et séché pour donner 48 mg de 6-[(E)-(hydroxyimino)méthyl]-*N-*phénylimidazo[1,2-*a*]pyridine-2-carboxamide sous la forme d'un solide blanc.

### Exemple 2 : 6-(Isopropylamino)-N-phénylimidazo[1,2-a]pyridine-1-carboxamide et son chlorhydrate (1:1).

### 2.1 : 5-Isopropylaminopyridine-2-amine

Après avoir ajouté lentement 1 g de chlorure stanneux à une suspension de 362 mg de 5-isopropylamino-6-nitro-pyridine (WO 2006040520) dans 10 mL d'éthanol, on chauffe le mélange réactionnel au reflux pendant 30 minutes puis concentre à sec. Le résidu est repris dans du méthanol ammoniacal, le mélange est filtré et le filtrat concentré à sec. Le résidu est chromatographié sur une cartouche de silice en éluant par un gradient de dichlorométhane et d'acétate d'éthyle (de 0/100 à 100/0) puis par un mélange de dichlorométhane et de méthanol ammoniacal 7N 90/10. Les fractions contenant le produit attendu sont réunies et concentrées à sec pour donner 143 mg de 5-isopropylaminopyridine-2-amine sous la forme d'une huile violette.

Spectre RMN ¹H (DMSO-d6, δ en ppm) : 1,08 (d, J = 6,5 Hz, 6H), 3,38 (m, 1H), 4,58 (m étalé, 1H), 5,18 (m étalé, 2H), 6,38 (d, J = 8,5 Hz, 1H), 6,89 (dd, J = 3,0 et 8,5 Hz, 1H), 7,33 (d, J = 3,0 Hz, 1H).

Spectre de masse (LC-MS-DAD-ELSD) : m/z 152 [M+H]⁺.

### 2.2 : 6-(Isopropylamino)-N-phénylimidazo[1,2-a]pyridine-2-carboxamide et son chlorhydrate (1:1)

A une solution de 143 mg de 5-isopropylaminopyridine-2-amine dans 10 mL de 1,2-diméthoxyéthane et 1 mL d'éthanol on ajoute 172 mg de 3-bromo-2-oxo-*N*-phényl-propionamide. Le mélange réactionnel est agité 90 heures à 25°C puis porté au reflux pendant 2 heures et concentré à sec sous pression réduite. Le résidu repris dans un mélange de dichlorométhane et d'une solution saturée de bicarbonate de sodium. La phase organique est séchée sur sulfate de magnésium et concentrée à sec sous pression réduite. Le résidu est chromatographié sur une cartouche de silice en éluant par un mélange de dichlorométhane et d'acétate d'éthyle (80/20). Les fractions contenant le produit attendu sont réunies et concentrées à sec pour donner 136 mg de 6-(isopropylamino)-*N*-phénylimidazo[1,2-*a*]pyridine-2-carboxamide sous la forme d'un solide brun.

Ce produit est repris dans du dioxanne additionné d'un peu de méthanol et traité par 116 uL d'une solution 4N d'acide chlorhydrique dans le dioxanne. Après 1 heure d'agitation à 20°C le précipité est essoré, lavé par du dioxanne et séché pour donner 136 mg de chlorhydrate (1:1) de 6-(isopropylamino)-*N*-phénylimidazo[1,2-*a*]pyridine-2-carboxamide sous la forme d'un solide rose pâle.

### Exemple 3 : 6-Nitro-N-phénylimidazo[1,2-a]pyridine-2-carboxamide

A une solution de 638 µL d'aniline dans 78 mL de toluène refroidie à 0°, on ajoute goutte à goutte 4,68 mL d'une solution de triméthylaluminium 2M dans le toluène puis à 20°C, 800 mg de 6-nitro-imidazo[1,2-a]pyridine-2-carboxylate d'éthyle (Heterocycles 38(7), 1527 (1994)). Le mélange réactionnel est agité 2 heures à la température ambiante puis refroidi à 4°C puis traité lentement par 40 mL d'une solution saturée de chlorure d'ammonium et concentré sous pression réduite et redilué par 150 mL d'eau et 400 mL d'acétate d'éthyle. La phase organique est séchée sur sulfate de magnésium, filtrée sur célite, évaporée à sec sous pression réduite. Le résidu est trituré avec du dichlorométhane, essoré et séché pour donner 247 mg de de 6-nitro-*N*-phénylimidazo[1,2-*a*]pyridine-2-carboxamide) sous la forme d'un solide gris vert.

### Exemple 4 : 6-Amino-N-phénylimidazo[1,2-a]pyridine-2-carboxamide

A une suspension de 190 mg de 6-nitro-*N*-phénylimidazo[1,2-*a*]pyridine-2-carboxamide dans 3 mL d'éthanol on ajoute 562 mg de chlorure stanneux et l'on chauffe au reflux pendant 30 minutes. Le mélange réactionnel est concentré à sec, le résidu est repris dans une solution d'ammoniac dans le méthanol (7N). La suspension est filtrée et le filtrat évaporé à sec sous pression réduite. Le résidu est chromatographié sur une cartouche de silice en éluant par un mélange de dichlorométhane et d'acétate d'éthyle 50/50. Les fractions contenant le produit attendu sont réunies et évaporées à sec sous pression réduite pour donner 30 mg de 6-amino-*N-*phénylimidazo[1,2-*a*]pyridine-2-carboxamide sous la forme d'un solide vert

La chromatographie permet également d'isoler les produits des exemples 5 et 6.

### Exemple 5 : 6-Amino-5-chloro-N-phénylimidazo[1,2-a]pyridine-2-carboxamide

10 mg de 6-amino-5-chloro-*N*-phénylimidazo[1,2-*a*]pyridine-2-carboxamide sont obtenus comme sous-produit de la préparation du composé de l'exemple 4. Le produit est isolé lors de la chromatographie sur silice sous la forme d'un solide vert.

### Exemple 6 : 6-Amino-5-éthoxy-N-phénylimidazo[1,2-a]pyridine-2-carboxamide

16 mg de 6-amino-5-éthoxy-*N*-phénylimidazo[1,2-*a*]pyridine-2-carboxamide sont obtenus comme sous-produit de la préparation du composé de l'exemple 4. Le produit est isolé lors de la chromatographie sur silice sous la forme d'un solide vert.

### Exemple 7 : 6-{[(1E)-(diméthylamino)méthylène]amino}-N-phénylimidazo[1,2-a]pyridine-2-carboxamide

### 7. 1 : 6-{[(1E)-(diméthylamino)méthylène]amino}-5-méthylimidazo[1,2-a]pyridine-2-carboxylate d'éthyle

On traite 200 mg de 6-amino-imidazo[1,2-*a*]pyridine-2-carboxylate d'éthyle (Heterocycles 38(7), 1527 (1994)) par 2 mL de diméthylacétal de *N*,*N-*diméthylformamide. Le mélange réactionnel est chauffé au reflux pendant 2 heures puis refroidi et dilué par du pentane. Le précipité est essoré, lavé et séché pour donner 180 mg de 6-{[(1E)-(diméthylamino)méthylène]amino}-5-méthylimidazo[1,2-*a*]pyridine-2-carboxylate d'éthyle sous la forme d'un solide ocre.

Spectre RMN ¹H (DMSO-d6, δ en ppm) : 1,31 (t, J = 3H), 2,91 (s large, 3H), 3,02 (s large, 3H), 4,29 (q, J = 7,0 Hz, 2H), 7,21 (dd, J = 1,5 et 9,5 Hz, 1H), 7,47 (d, J = 9,5 Hz, 1H), 7,84 (s, 1H), 8,05 (d, J = 1,5 Hz, 1H), 8,35 (s, 1H).

Spectre de masse (LC-MS-DAD-ELSD) : m/z 261 [M+H]⁺, m/z 233 [MH-C₂H₅]⁺

### 7.2 : 6-{[(1E)-(diméthylamino)méthylène]amino}-N-phénylimidazo[1,2-a]pyridine-2-carboxamide

En opérant de manière analogue à l'exemple 2 (étape 2.2) on obtient à partir de 112 mg de 6-{[(1E)-(diméthylamino)méthylène]amino}-5-méthylimidazo[1,2-a]pyridine-2-carboxylate d'éthyle, 79 mg de 6-{[(1E)-(diméthylamino)méthylène]amino}-*N*-phénylimidazo[1,2-a]pyridine-2-carboxamide sous la forme d'un solide beige.

Les intermédiaires décrits ci-dessous sont utiles à la préparation des composés de la présente invention.

### 5-Ethylaminopyridine-2-amine

La 5-éthylaminopyridine-2-amine est préparée de la même manière que la 5-isopropylamino-pyridine-2-amine (exemple 2.1) à partir de la 5-éthylamino-6-nitro-pyridine (PCT Int. Appl. WO 2006040520).

### 5-(2-Méthoxyéthylamino)pyridine-2-amine

370 mg de 5-(2-méthoxyéthylamino)-2-nitropyridine (WO 2006040526) sont ajoutés lentement à une suspension de 597 mg d'étain dans 6 mL d'acide bromhydrique à 48 % refroidie à -5°C. Le mélange réactionnel est agité 2 heures à -5°C. On ajoute lentement 6 mL d'ammoniaque à 25 % et extrait par du dichlorométhane. La phase organique est décantée, séchée sur sulfate de magnésium et concentrée à sec sous pression réduite pour donner la 5-(2-méthoxyéthylamino)pyridine-2-amine brute sous la forme d'une huile brune qui est utilisée tel quelle pour la suite de la synthèse.

Spectre de masse (LC/MS) : m/z 168 : [M+H]⁺.

Les tableaux qui suivent illustrent les structures chimiques (tableau 1) et les caractéristiques spectroscopiques (tableau 2) de quelques exemples de composés selon l'invention.

Dans ce tableaux, « HCl » signifie chlorhydrate ; « HBr » signifie bromhydrate ; « - » signifie que le composé se présente sous forme base ; « Me » signifie un groupe méthyle, « Et » signifie un groupe éthyle, « iPr » signifie un groupe isopropyle, « OMe » signifie un groupe méthoxy.

**Tableau 1**

| | | | | | | |
|---|---|---|---|---|---|---|
| 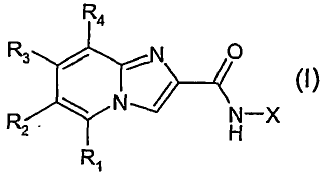 | | | | | | |

| **Ex** | **R₁** | **R₂** | **R₃** | **R₄** | **X** | **sel** |
|---|---|---|---|---|---|---|
| **1** | H | CH=NOH | H | H | Ph | - |
| **2** | H | NHiPr | H | H | Ph | HCl |
| **3** | H | NO₂ | H | H | Ph | - |
| **4** | H | NH₂ | H | H | Ph | - |
| **5** | Cl | NH₂ | H | H | Ph | - |
| **6** | OEt | NH₂ | H | H | Ph | - |
| **7** | H | N=CHNMe₂ | H | H | Ph | - |
| **8** | H | CH=NOMe | H | H | Ph | - |
| **9** | H | NHMe | H | H | Ph | - |
| **10** | H | NHEt | H | H | Ph | - |
| **11** | H | NHCH₂CH₂OMe | H | H | Ph | - |
| **12** | Me | NO₂ | H | H | Ph | - |
| **13** | Me | NH₂ | H | H | Ph | - |
| **14** | H | SMe | H | H | Ph | HBr |
| **15** | H | SOMe | H | H | Ph | HBr |
| **16** | H | SO₂Me | H | H | Ph | HBr |
| **17** | H | SO₂NH₂ | H | H | Ph | HBr |
| **18** | H | SO₂NHMe | H | H | Ph | HBr |
| **19** | H | SO₂NMe₂ | H | H | Ph | - |
| **20** | H | C≡CSiMe₃ | H | H | Ph | HBr |

**Tableau 2**

| **Ex** | **Caractérisations** |
|---|---|
| **1** | Spectre RMN ¹H (DMSO-d6, δ en ppm) : 7,09 (t J = 7,5 Hz, 1H), 7,34 (t, J = 7,5 Hz, 2H), de 7,62 à 7,72 (m, 2H), 7,89 (d, J = 7,5 Hz, 2H), 8,20 (s, 1H), 8,55 (s, 1H), 8,79 (s large, 1H), 10,2 (s, 1H), 11,45 (s large, 1H). Spectre de masse (LC-MS-DAD-ELSD) : m/z 281 [M+H]⁺. |
| **2** | Spectre RMN 1H (DMSO-d6, δ en ppm) : 1,20 (d, J = 6,5 Hz, 6H), 3,47 (m, 1H), 7,14 (t, J = 8,0 Hz, 1H), 7,35 (m partiellement masqué, 1H), 7,39 (t, J = 8,0 Hz, 2H), 7,58 (d, J = 9,5 Hz, 1H), 7,81 (d, J = 8,0 Hz, 2H), 7,92 (s large, 1H), 8,59 (s large, 1H), 10,6 (m étalé, 1H). Spectre de masse (LC-MS-DAD-ELSD) : m/z 295 [M+M]⁺. |
| **3** | Spectre RMN 1H (DMSO-d6, δ en ppm) : 7,11 (t, J = 8,0 Hz, 1H), 7,36 (t, J = 8,0 Hz, 2H), 7,82 (d, J = 9,5 Hz, 1H), 7,89 (d, J = 8,0 Hz, 2H), 8,08 (dd, J = 2,0 et 9,5 Hz, 1H), 8,74 (s, 1H), 9,96 (d, J = 2,0 Hz, 1H), 10,4 (s large, 1H). Spectre de masse (LC-MS-DAD-ELSD): m/z 283 [M+H]⁺. |
| **4** | Spectre RMN 1H (DMSO-d6, δ en ppm) : 5,10 (s large, 2H), 6,99 (dd, J = 1,5 et 9,5 Hz, 1H), 7,06 (t, J = 7,5 Hz, 1H), 7,31 (t, J = 7,5 Hz, 2H), 7,41 (d, J = 9,5 Hz, 1H), 7,71 (d, J = 1,5 Hz, 1H), 7,87 (d, J = 7,5 Hz, 2H), 8,29 (s, 1H), 10,05 (s, 1H). Spectre de masse (LC-MS-DAD-ELSD): m/z 252 [M]⁺. |
| **5** | Spectre RMN 1H (DMSO-d6, δ en ppm) : 5,50 (s, 2H), 7,09 (t, J = 7,5 Hz, 1H), 7,20 (d, J = 9,5 Hz, 1H), 7,32 (t, J = 7,5 Hz, 2H), 7,53 (d, J = 9,5 Hz, 1H), 7,88 (d, J = 7,5 Hz, 2H), 8,19 (s, 1H), 10,15 (s, 1H). Spectre de masse (LC-MS-DAD-ELSD) : m/z 287 [M+H]⁺, présence de 1 Cl. |
| **6** | Spectre RMN 1H (DMSO-d6, δ en ppm) : 1,43 (t, J = 7,0 Hz, 3H), 4,19 (q, J = 7,0 Hz, 2H), 4,82 (s large, 2H), 7,07 (t, J = 7,5 Hz, 1H), 7,15 (d, J = 9,0 Hz, 1H), 7,31 (d, J = 9,0 Hz, 1H), 7,33 (t, J = 7,5 Hz, 2H), 7,89 (d, J = 7,5 Hz, 2H), 8,12 (s, 1H), 10,1 (s, 1H). Spectre de masse (LC-MS-DAD-ELSD) : m/z 297 [M+H]⁺. |
| **7** | Spectre RMN 1H (DMSO-d6, δ en ppm) : 2,93 (s large, 3H), 3,03 (s large, 3H), 7,09 (t, J = 7,5 Hz, 1H), 7,24 (dd, J = 1,5 et 9,5 Hz, 1H), 7,33 (t, J = 7,5 Hz, 2H), 7,51 (d, J = 9,5 Hz, 1H), 7,87 (s, 1H), 7,89 (d, J = 7,5 Hz, 2H), 8,11 (s large, 1H), 8,34 (s, 1H), 10,1 (s, 1H). Spectre de masse (LC-MS-DAD-ELSD) : m/z 308 [M+H]⁺. |
| **8** | Spectre RMN 1H (DMSO-d6, δ en ppm) : 3,92 (s, 3H), 7,10 (t J = 7,5 Hz, 1H), 7,34 (t, J = 7,5 Hz, 2H), de 7,62 à 7,72 (m, 2H), 7,89 (d, J = 7,5 Hz, 2H), 8,30 (s, 1H), 8,59 (s, 1H), 8,83 (s large, 1H), 10,25 (s large, 1H). Spectre de masse (LC-MS-DAD-ELSD) : m/z 295 [M+M]⁺. |
| **9** | Spectre RMN 1H (DMSO-d6, δ en ppm) : 2,68 (d, J = 5,5 Hz, 3H), 5,72 (q, J =5,5 Hz, 1H), 6,99 (dd, J = 1,5 et 9,5 Hz, 1H), 7,08 (t, J = 7,5 Hz, 1H), 7,32 (t, J = 7,5 Hz, 2H), 7,41 (d, J = 9,5 Hz, 1H), 7,58 (d, J = 1,5 Hz, 1H), 7,87 (d large, J = 7,5 Hz, 2H), 8,29 (s, 1H), 10,0 (s, 1H). Spectre de masse (LC-MS-DAD-ELSD) : m/z 267 [M+H]⁺. |
| **10** | Spectre RMN 1H (DMSO-d6, δ en ppm) : 1,22 (t, J = 7,5 Hz, 3H), 2,98 (m, 2H), 5,62 (t, J = 5,5 Hz, 1H), 7,00 (dd, J = 1,5 et 9,5 Hz, 1H), 7,07 (t large, J = 7,5 Hz, 1H), 7,31 (t, J = 7,5 Hz, 2H), 7,42 (d, J = 9,5 Hz, 1H), 7,61 (d, J = 1,5 Hz, 1H), 7,87 (d, J = 7,5 Hz, 2H), 8,27 (s, 1H), 10,0 (s, 1H). Spectre de masse (LC-MS-DAD-ELSD) : m/z 281 [M+H]⁺. |
| **11** | Spectre RMN 1H (DMSO-d6, δ en ppm) : 3,12 (q, J = 6,0 Hz, 2H), 3,30 (s masqué, 3H), 3,58 (t, J = 6,0 Hz, 2H), 5,70 (t, J = 6,0 Hz, 1H), 7,07 (m, 2H), 7,31 (t, J = 7,5 H, 2H), 7,42 (d, J = 9,5 Hz, 1H), 7,69 (d, J = 1,5 Hz, 1H), 7,88 (d, J = 7,5 Hz, 2H), 8,25 (s, 1H), 10,0 (s, 1H). Spectre de masse (LC-MS-DAD-ELSD) : m/z 311 [M+H]⁺. |
| **12** | Spectre RMN 1H (DMSO-d6, δ en ppm) : 3,02 (s, 3H), 7,11 (t, J = 7,5 Hz, 1H), 7,37 (t, J = 7,5 Hz, 2H), 7,73 (d, J = 9,5 Hz, 1H), 7,90 (d, J = 7,5 Hz, 2H), 7,97 (d, J = 9,5 Hz, 1H), 8,81 (s, 1H), 10,4 (s, 1H). Spectre de masse (LC-MS-DAD-ELSD) : m/z 295 [M-H]⁻, m/z 297 [M+H]⁺. |
| **13** | Spectre RMN 1H (DMSO-d6, δ en ppm) : 2,41 (s, 3H), 4,93 (s large, 2H), 7,08 (m, 2H), 7,32 (t, J = 7,5 Hz, 2H), 7,38 (d, J = 9,5 Hz, 1H), 7,89 (d, J = 7,5 Hz, 2H), 8,13 (s, 1H), 10,1 (s, 1H). Spectre de masse (LC-MS-DAD-ELSD) : m/z 267 [M+H]⁺, m/z = 192 [MH-Ph]⁺, m/z = 174 [MH-NHPh]⁺. |
| **14** | Spectre RMN 1H (DMSO-d6, δ en ppm) : 2,58 (s, 3H), 7,12 (t, J = 8,0 Hz, 1H), 7,38 (t, J = 8,0 Hz, 2H), 7,58 (d large, J = 9,5 Hz, 1H), 7,69 (d, J = 9,5 Hz, 1H), 7,85 (d, J = 8,0 Hz, 2H), 8,59 (s, 1H), 8,69 (s large, 1H), 10,5 (s, 1H). Spectre de masse (LC-MS-DAD-ELSD) : m/z 283 [M]⁺. |
| **15** | Spectre RMN 1H (DMSO-d6, δ en ppm) : 2,90 (s, 3H), 7,11 (t, J = 7,5 Hz, 1H), 7,36 (t, J = 7,5 Hz, 2H), 7,69 (d large, J = 9,5 Hz, 1H), 7,84 (d, J = 9,5 Hz, 1H), 7,89 (d, J = 7,5 Hz, 2H), 8,70 (s, 1H), 9,04 (s large, 1H), 10,35 (s, 1H). Spectre de masse (LC-MS-DAD-ELSD) : m/z 298 [M-H]⁻, m/z 300 [M+H]⁺. |
| **16** | Spectre RMN 1H (DMSO-d6, δ en ppm) : 3,36 (s, 3H), 7,11 (t, J = 7,5 Hz, 1H), 7,35 (t, J = 7,5 Hz, 2H), 7,78 (d large, J = 9,5 Hz, 1H), 7,89 (m, 3H), 8,75 (s, 1H), 9,39 (s large, 1H), 10,4 (s, 1H). Spectre de masse (LC-MS-DAD-ELSD) : m/z 314 [M-H]⁻, m/z 316 [M+H]⁺. |
| **17** | Spectre RMN 1H (DMSO-d6, δ en ppm) : 7,11 (t, J = 7,5 Hz, 1H), 7,35 (t, J = 7,5 Hz, 2H), 7,67 (s large, 2H), 7,69 (d large, J = 9,5 Hz, 1H), 7,86 (d, J = 9,5 Hz, 1H), 7,89 (d, J = 7,5 Hz, 2H), 8,73 (s, 1H), 9,26 (s large, 1H), 10,35 (s, 1H). Spectre de masse (LC-MS-DAD-ELSD) : m/z 315 [M-H]⁻, m/z 317 [M+H]⁺. |
| **18** | Spectre RMN 1H (DMSO-d6, δ en ppm) : 2,50 (m masqué, 3H), 7,11 (t, J = 7,5 Hz, 1H), 7,36 (t, J = 7,5 Hz, 2H), 7,61 (d large, J = 9,5 Hz, 1H), 7,80 (q large, J = 5,5 Hz, 1H), 7,88 (m, 3H), 8,71 (s, 1H), 9,27 (s large, 1H), 10,4 (s, 1H). Spectre de masse (LC-MS-DAD-ELSD) : m/z 329 [M-H]⁻, m/z 331 [M+H]⁺. |
| **19** | Spectre RMN 1H (DMSO-d6, δ en ppm) : 2,74 (s, 6H), 7,11 (t, J = 7,5 Hz, 1H), 7,36 (t, J = 7,5 Hz, 2H), 7,58 (dd, J = 1,5 et 9,5 Hz, 1H), 7,82 (d, J = 9,5 Hz, 1H), 7,90 (d, J = 7,5 Hz, 2H), 8,69 (s, 1H), 9,30 (s large, 1H), 10,35 (s, 1H). Spectre de masse (LC-MS-DAD-ELSD) : m/z 343 [M-H]⁻, m/z 345 [M+H]⁺. |
| **20** | Spectre RMN 1H (DMSO-d6, δ en ppm) : 0,27 (s, 9H), 7,12 (t, J=7,7 Hz, 1H), 7,36 (t, J=7,7 Hz, 2H), 7,47 (dd, J=9,3, 1,5 Hz, 1H), 7,68 (d, J=9,3 Hz, 1H), 7,87 (d large, J=7,8 Hz, 2H), 8,54 (s, 1H), 9,02 (s large, 1H), 10,41 (s, 1 H). Spectre de masse (LC-MS-DAD-ELSD) : m/z 334 [M+H]⁺. |

Les composés selon l'invention ont fait l'objet d'essais pharmacologiques permettant de déterminer leur effet modulateur sur NOT

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 1000 nM. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Par exemple, les composés n° 2, 14 et 16 ont montré une CE₅₀ de respectivement 1,6 nM, 2 nM et 16 nM.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcéreuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées ; l'ostéoporose ; les cancers.

Ainsi, la présente invention vise un composé choisi parmi les composés de formule (I) définis précédemment, pour le traitement et la prévention de l'une des maladies précitées.

Selon un mode de réalisation particulier, ces médicaments trouvent leur emploi dans le traitement et la prévention de l'une des maladies précitées, à l'exception des maladies inflammatoires.

Selon un autre de ses aspects, la présente invention concerne l'utilisation d'un composé choisi parmi les composés de formule (I) tels que définis précédemment, pour la préparation d'un médicament destiné au traitement ou à la prévention de l'une des maladies mentionnées ci-dessous.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants:

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composés de formule (I) : 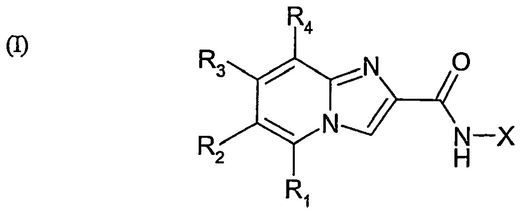 dans laquelle :
X représente un groupe phényle éventuellement substitué par un ou plusieurs groupes choisis indépendamment les uns des autres parmi les atomes ou groupes suivants : halogène, (C₁-C₆)alcoxy, (C₁-C₆)alkyle, NRaRb, les groupes alkyle et alcoxy pouvant éventuellement être substitués par un ou plusieurs atomes d'halogène ;
R₁ représente un atome d'hydrogène, un atome d'halogène, un groupe (C₁-C₆)alcoxy, un groupe (C₁-C₆)alkyle, un amino, un groupe NRcRd; les groupes alkyle et alcoxy pouvant éventuellement être substitués par un ou plusieurs atomes d'halogènes, un groupe hydroxy, amino, ou groupe (C₁-C₆)alcoxy ;
R₂ représente l'un des groupes suivants :
. un groupe NRcRd,
. un groupe -N=CH-NRaRb,
. nitro, hydroxyiminoalkyle, alcoxyiminoalkyle,
. un groupe (C₁-C₆)alkylthio,
. un groupe (C₁-C₆)alkylsulfinyle,
. un groupe (C₁-C₆)akylsulfonyle,
. un groupe -SO₂-NR₅R₆,
. un groupe (((C₁-C₆)alkyl)₃)silyléthynyle ;
R₃ représente un atome d'hydrogène, un groupe (C₁-C₆)alkyle, un groupe (C₁-C₆)alcoxy ou un atome d'halogène ;
R₄ représente un atome d'hydrogène, un groupe (C₁-C₄)alkyle, un groupe (C₁-C₄)alcoxy ou un atome de fluor ;
R₅ et R₆, identiques ou différents, représentent un atome d'hydrogène ou un groupe (C₁-C₆)alkyle ;
Ra et Rb représentent, indépendamment l'un de l'autre, un atome d'hydrogène, un groupe (C₁-C₆)alkyle ou forment avec l'atome d'azote qui les porte un cycle de 4 à 7 chaînons ;
Rc représente un atome d'hydrogène et Rd représente un atome d'hydrogène ou un groupe
(C₁-C₆)alkyle, ledit alkyle étant éventuellement substitué par un groupe (C₁-C₆) alcoxy ; à l'état de base ou de sel d'addition à un acide.

2. Composés de formule (I) selon la revendication 1, **caractérisé en ce que** l'un des groupements R₁ ou R₂ est différent d'un atome d'hydrogène.

3. Composés de formule (I) selon la revendication 1 ou 2, **caractérisé en ce que** R₁ représente un atome d'hydrogène, un atome d'halogène, un groupe (C₁-C₆)alcoxy.

4. Composés de formule (I) selon l'une quelconque des revendications précédentes, **caractérisé en ce que** R₃ et R₄ représentent chacun un atome d'hydrogène.

5. Composés de formule (I) selon l'une quelconque des revendications précédentes, **caractérisé en ce que** R₂ représente l'un des groupes suivants :
. un groupe NRcRd,
. un groupe -N=CH-NRaRb,
. nitro, hydroxyiminoalkyle, alcoxyiminoalkyle,
. un groupe (C₁-C₆)alkylthio,
. un groupe (C₁-C₆)alkylsulfinyle,
. un groupe (C₁-C₆)alkylsulfonyle,
. un groupe -SO₂-NR₅R₆,
. un groupe (((C₁-C₆)alkyl)₃)silyléthynyle ;
R₅ et R₆, identiques ou différents, représentent un atome d'hydrogène ou un groupe (C₁-C₆)alkyle ; Ra et Rb représentent, indépendamment l'un de l'autre, un atome d'hydrogène ou un groupe (C₁-C₆)alkyle ;
Rc représente un atome d'hydrogène et Rd représente un atome d'hydrogène ou un groupe (C₁ - C₆)alkyle, ledit alkyle étant éventuellement substitué par un groupe (C₁-C₆) alcoxy.

6. Composés de formule (I) selon l'une quelconque des revendications précédentes, **caractérisé en ce que** :
X représente un groupe phényle ;
R₁ représente un atome d'hydrogène, un atome de chlore, un groupe méthyle ou éthoxy ;
R₃ et R₄ représentent un atome d'hydrogène ;
R₂ représente un groupe NH₂, CH=NOH, NHiPr, nitro, CH=NOMe, NHMe, N=CHNMe₂, NHEt, NHCH₂CH₂OMe, SMe, SOMe, SO₂Me, SO₂NH₂, SO₂NHMe, SO₂NMe₂, C≡CSiMe₃ ; à l'état de base ou de sel d'addition à un acide.

7. Composé selon l'une quelconque des revendications précédentes, **caractérisé en ce qu'**il est choisi parmi :
6-[(E)-(hydroxyimino)méthyl]-*N*-phénylimidazo[1,2-*a*]pyridine-2-carboxamide
6-(Isopropylamino)-*N*-phénylimidazo[1,2-*a*]pyridine-2-carboxamide et son chlorhydrate (1:1)
6-Nitro-*N*-phénylimidazo[1,2-*a*]pyridine-2-carboxamide
6-Amino*-N*-phénylimidazo[1,2-*a*]pyridine-2-carboxamide
6-Amino-5-chloro-*N*-phénylimidazo[1,2-*a*]pyridine-2-carboxamide
6-Amino-5-éthoxy-*N*-phénylimidazo[1,2-*a*]pyridine-2-carboxamide
6-{[(1E)-(diméthylamino)méthylène]amino}-*N*-phénylimidazo[1,2-*a*]pyridine-2-carboxamide
6-[(E)-(méthoxyimino)méthyl]-*N*-phénylimidazo[1,2-*a*]pyridine-2-carboxamide
6-(Méthylamino)-*N*-phénylimidazo[1,2-*a*]pyridine-2-carboxamide
6-(Ethylamino)-*N*-phénylimidazo[1,2-*a*]pyridine-2-carboxamide
6-(2-Méthoxyéthylamino)-*N*-phénylimidazo[1,2-*a*]pyridine-2-carboxamide
5-Méthyl-6-nitro-*N*-phénylimidazo[1,2-*a*]pyridine-2-carboxamide
6-Amino-5-méthyl-*N*-phénylimidazo[1,2-*a*]pyridine-2-carboxamide
6-(Méthylthio)-*N*-phénylimidazo[1,2-*a*]pyridine-2-carboxamide et son bromhydrate (1:1)
6-[(RS)-méthylsulfinyl]-*N*-phénylimidazo[1,2-*a*]pyridine-2-carboxamide et son bromhydrate (1:1)
6-(Méthylsulfonyl)-*N*-phénylimidazo[1,2-*a*]pyridine-2-carboxamide et son bromhydrate (1:1)
6-(Aminosulfonyl)-*N*-phénylimidazo[1,2-*a*]pyridine-2-carboxamide et son bromhydrate (1:1)
6-[(Méthylamino)sulfonyl]-*N*-phénylimidazo[1,2-*a*]pyridine-2-carboxamide et son bromhydrate (1:1)
6-[(Diméthylamino)sulfonyl]-*N*-phénylimidazo[1,2-*a*]pyridine-2-carboxamide
6-(Triméthylsilyléthynyl)-*N*-phénylimidazo[1,2-*a*]pyridine-2-carboxamide et son bromhydrate (1:1)
et un sel d'addition de ces composés à un acide pharmaceutiquement acceptable.

8. Médicament, **caractérisé en ce qu'**il comprend un composé de formule (I) selon l'une quelconque des revendications 1 à 7, ou un sel d'addition de ce composé à un acide pharmaceutiquement acceptable.

9. Composition pharmaceutique, **caractérisée en ce qu'**elle comprend un composé de formule (I) selon l'une quelconque des revendications 1 à 7, ou un sel pharmaceutiquement acceptable, de ce composé, ainsi qu'au moins un excipient pharmaceutiquement acceptable.

10. Utilisation d'un composé de formule (I) selon l'une quelconque des revendications 1 à 7 pour la préparation d'un médicament destiné au traitement et à la prévention des maladies neurodégénératives.

11. Utilisation d'un composé de formule (I) selon l'une quelconque des revendications 1 à 7 pour la préparation d'un médicament destiné au traitement et à la prévention des traumatismes cérébraux et de l'épilepsie.

12. Utilisation d'un composé de formule (I) selon l'une quelconque des revendications 1 à 7 pour la préparation d'un médicament destiné au traitement et à la prévention des maladies psychiatriques.

13. Utilisation d'un composé de formule (I) selon l'une quelconque des revendications 1 à 7 pour la préparation d'un médicament destiné au traitement et à la prévention des maladies inflammatoires.

14. Utilisation d'un composé de formule (I) selon l'une quelconque des revendications 1 à 7 pour la préparation d'un médicament destiné au traitement et à la prévention de l'ostéoporose.

15. Utilisation d'un composé de formule (I) selon l'une quelconque des revendications 1 à 7 pour la préparation d'un médicament destiné au traitement et à la prévention des cancers.

16. Utilisation d'un composé de formule (I) selon l'une quelconque des revendications 1 à 7 pour la préparation d'un médicament destiné au traitement et à la prévention de la maladie de Parkinson, d'Alzheimer, des tauopathies, de la sclérose en plaque.

17. Utilisation d'un composé de formule (I) selon l'une quelconque des revendications 1 à 7 pour la préparation d'un médicament destiné au traitement et à la prévention de la schizophrénie, la dépression, la dépendance à une substance les troubles du déficit de l'attention et de l'hyperactivité.

18. Composés intermédiaires
5-Ethylaminopyridine-2-amine
5-(2-Méthoxyéthylamino)pyridine-2-amine.

19. Utilisation des composés selon la revendication 18 pour la préparation des composés tels que définis dans les revendications 1 à 7.

## Patentansprüche

1. Verbindungen der Formel (I): 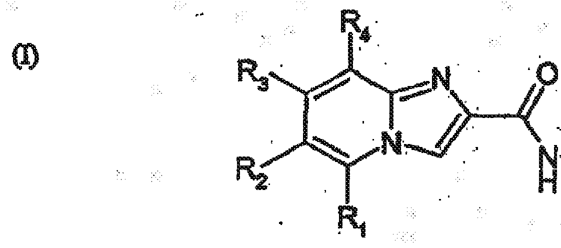 worin
X für eine Phenylgruppe steht, die gegebenenfalls durch eine oder mehrere Gruppen substituiert ist, die unabhängig voneinander unter den folgenden Atomen bzw. Gruppen ausgewählt sind: Halogen, (C₁-C₆)-Alkoxy, (C₁-C₆)-Alkyl und NRaRb, wobei die Alkyl- und Alkoxygruppen gegebenenfalls durch ein oder mehrere Halogenatome substituiert sein können;
R₁ für ein Wasserstoffatom, ein Halogenatom, eine (C₁-C₆)-Alkoxygruppe, eine (C₁-C₆)-Alkylgruppe, ein Amino oder eine NRcRd-Gruppe steht; wobei die Alkyl- und Alkoxygruppen gegebenenfalls durch ein oder mehrere Halogenatome oder eine Hydroxy-, Amino- oder (C₁-C₆)-Alkoxygruppe substituiert sein können;
R₂ für eine der folgenden Gruppen steht:
eine NRcRd-Gruppe,
. eine - N =CH-NRaRb-Gruppe,
. Nitro, Hydroxyiminoalkyl, Alkoxyiminoalkyl,
. eine (C₁-C₆)-Alkylthiogruppe,
. eine (C₁-C₆)-Alkylsulfinylgruppe,
. eine (C₁-C₆)-Alkylsulfonylgruppe,
. eine -SO₂-NR₅R₆-Gruppe,
. eine (((C₁-C₆)-Alkyl)₃)silylethinylgruppe;
R₃ für ein Wasserstoffatom, eine (C₁-C₆)-Alkyl gruppe, eine (C₁-C₆)-Alkoxygruppe oder ein Halogenatom steht;
R₄ für ein Wasserstoffatom, eine (C₁-C₄)-Alkylgruppe, eine (C₁-C₄)-Alkoxygruppe oder ein Fluoratom steht;
R₅ und R₆ gleich oder verschieden sind und für ein Wasserstoffatom oder eine (C₁-C₆)-Alkylgruppe stehen;
Ra und Rb unabhängig voneinander für ein Wasserstoffatom oder eine (C₁-C₆)-Alkylgruppe stehen oder mit dem Stickstoffatom, an das sie gebunden sind, einen 4- bis 7-gliedrigen Ring bilden;
Rc für ein Wasserstoffatom steht und Rd für ein Wasserstoffatom oder eine (C₁-C₆)-Alkylgruppe steht, wobei das Alkyl gegebenenfalls durch eine (C₁-C₆)-Alkoxygruppe substituiert sein kann;
in Basen- oder Säureadditionssalzform.

2. Verbindungen der Formel (I) nach Anspruch 1, **dadurch gekennzeichnet, daß** eine der Gruppen R₁ und R₂ von einem Wasserstoffatom verschieden ist.

3. Verbindungen der Formel (I) nach Anspruch 1 oder 2, **dadurch gekennzeichnet, daß** R₁ für ein Wasserstoffatom, ein Halogenatom oder eine (C₁-C₆)-Alkoxygruppe steht.

4. Verbindungen der Formel (I) nach einem der vorhergehenden Ansprüche, **dadurch gekennzeichnet, daß** R₃ und R₄ jeweils für ein Wasserstoffatom stehen.

5. Verbindungen der Formel (I) nach einem der vorhergehenden Ansprüche, **dadurch gekennzeichnet, daß** R₂ für eine der folgenden Gruppen steht:
. eine NRcRd-Gruppe,
. eine - N =CH-NRaRb-Gruppe,
. Nitro, Hydroxyiminoalkyl, Alkoxyiminoalkyl,
. eine (C₁-C₆)-Alkylthiogruppe,
. eine (C₁-C₆)-Alkylsulfinylgruppe,
. eine (C₁-C₆)-Alkylsulfonylgruppe,
. eine -SO₂-NR₅R₆-Gruppe,
. eine (((C₁-C₆)-Alkyl)₃)silylethinylgruppe;
R₅ und R₆ gleich oder verschieden sind und für ein Wasserstoffatom oder eine (C₁-C₆)-Alkylgruppe stehen;
Ra und Rb unabhängig voneinander für ein Wasserstoffatom oder eine (C₁-C₆)-Alkylgruppe stehen;
Rc für ein Wasserstoffatom steht und Rd für ein Wasserstoffatom oder eine (C₁-C₆)-Alkylgruppe steht, wobei das Alkyl gegebenenfalls durch eine (C₁-C₆)-Alkoxygruppe substituiert sein kann.

6. Verbindungen der Formel (I) nach einem der vorhergehenden Ansprüche, **dadurch gekennzeichnet, daß**:
X für eine Phenylgruppe steht;
R₁ für ein Wasserstoffatom, ein Chloratom oder eine Methyl- oder Ethoxygruppe steht;
R₃ und R₄ für ein Wasserstoffatom stehen;
R₂ für eine NH₂-, CH=NOH-, NHiPr-, Nitro-, CH=NOMe-, NHMe-, N=CHNMe₂-, NHEt-, NHCH₂CH₂OMe-SMe-, SOMe-, SO₂Me, SO₂NH₂-, SO₂NHMe-, SO₂NMe₂- oder C≡CSiMe₃-Gruppe steht;
in Basen- oder Säureadditionssalzform.

7. Verbindung nach einem der vorhergehenden Ansprüche, **dadurch gekennzeichnet, daß** sie ausgewählt ist unter:
6-[(E)-(Hydroxyimino)methyl]-*N*-phenylimidazo-[1,2-α]pyridin-2-carboxamid
6-(Isopropylamino)-*N*-phenylimidazo[1,2-α]pyridin-2-carboxamid und dessen Hydrochlorid (1:1)
6-Nitro-*N*-phenylimidazo[1,2-α]pyridin-2-carboxamid
6-Amino-*N*-phenylimidazo[1,2-α]pyridin-2-carboxamid
6-Amino-5-chloro-*N*-phenylimidazo[1,2-α]pyridin-2-carboxamid
6-Amino-5-ethoxy-*N*-phenylimidazo[1,2-α]pyridin-2-carboxamid
6-{[(1E)-(Dimethylamino)methylen]amino}-*N*-phenylimidazo[1,2-α]pyridin-2-carboxamid
6-[(E)-(Methoxyimino)methyl]-*N*-phenylimidazo[1,2-α]pyridin-2-carboxamid
6-(Methylamino)-*N*-phenylimidazo[1,2-α]pyridin-2-carboxamid
6-(Ethylamino)-*N*-phenylimidazo[1,2-α]pyridin-2-carboxamid
6-(2-Methoxyethylamino)-*N*-phenylimidazo[1,2-α]-pyridin-2-carboxamid
5-Methyl-6-nitro-*N*-phenylimidazo[1,2-α]pyridin-2-carboxamid
6-Amino-5-methyl-*N*-phenylimidazo[1,2-α]pyridin-2-carboxamid
6-(Methylthio)-*N*-phenylimidazo[1,2-α]pyridin-2-carboxamid und dessen Hydrobromid (1:1)
6-[(RS)-Methylsulfinyl]-*N*-phenylimidazo[1,2-α]-pyridin-2-carboxamid und dessen Hydrobromid (1:1)
6-(Methylsulfonyl)-*N*-phenylimidazo[1,2-α]pyridin-2-carboxamid und dessen Hydrobromid (1:1)
6-(Aminosulfonyl)-*N*-phenylimidazo[1,2-α]pyridin-2-carboxamid und dessen Hydrobromid (1:1)
6-[(Methylamino)sulfonyl]-*N*-phenylimidazo[1,2-α]-pyridin-2-carboxamid und dessen Hydrobromid (1:1)
6-[(Dimethylamino)sulphonyl]-*N*-phenylimidazo[1,2-*α*]pyridin-2-carboxamid
6-(Trimethylsilylethinyl)-*N*-phenylimidazo[1,2-α]-pyridin-2-carboxamid und dessen Hydrobromid (1:1) und einem Additionssalz dieser Verbindungen mit einer pharmazeutisch unbedenklichen Säure

8. Medikament, **dadurch gekennzeichnet, daß** es eine Verbindung der Formel (I) nach einem der Ansprüche 1 bis 7 oder ein Additionssalz dieser Verbindung mit einer pharmazeutisch unbedenklichen Säure enthält.

9. Pharmazeutische Zusammensetzung, **dadurch gekennzeichnet, daß** sie eine Verbindung der Formel (I) nach einem der Ansprüche 1 bis 7 oder ein pharmazeutisch unbedenkliches Salz dieser Verbindung sowie mindestens einen pharmazeutisch unbedenklichen Hilfsstoff enthält.

10. Verwendung einer Verbindung der Formel (I) nach einem der Ansprüche 1 bis 7 zur Herstellung eines Medikaments zur Behandlung und Prävention von neurodegenerativen Erkrankungen.

11. Verwendung einer Verbindung der Formel (I) nach einem der Ansprüche 1 bis 7 zur Herstellung eines Medikaments zur Behandlung und Prävention von Hirntraumen und Epilepsie.

12. Verwendung einer Verbindung der Formel (I) nach einem der Ansprüche 1 bis 7 zur Herstellung eines Medikaments zur Behandlung und Prävention von psychiatrischen Erkrankungen.

13. Verwendung einer Verbindung der Formel (I) nach einem der Ansprüche 1 bis 7 zur Herstellung eines Medikaments zur Behandlung und Prävention von entzündlichen Erkrankungen.

14. Verwendung einer Verbindung der Formel (I) nach einem der Ansprüche 1 bis 7 zur Herstellung eines Medikaments zur Behandlung und Prävention von Osteoporose.

15. Verwendung einer Verbindung der Formel (I) nach einem der Ansprüche 1 bis 7 zur Herstellung eines Medikaments zur Behandlung und Prävention von Krebserkrankungen.

16. Verwendung einer Verbindung der Formel (I) nach einem der Ansprüche 1 bis 7 zur Herstellung eines Medikaments zur Behandlung und Prävention von Parkinson-Krankheit, Alzheimer-Krankheit, Tauopathien und multipler Sklerose.

17. Verwendung einer Verbindung der Formel (I) nach einem der Ansprüche 1 bis 7 zur Herstellung eines Medikaments zur Behandlung und Prävention von Schizophrenie, Depression, Substanzabhängigkeit und Aufmerksamkeitsdefizit-Hyperaktivitätssyndrom.

18. Zwischenverbindungen
5-Ethylaminopyridin-2-amin
5-(2-Methoxyethylamino)pyridin-2-amin.

19. Verwendung der Verbindungen nach Anspruch 18 zur Herstellung von Verbindungen gemäß einem der Ansprüche 1 bis 7.

## Claims

1. Compounds of formula (I): 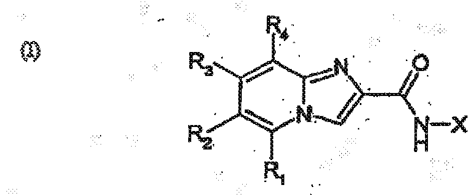 in which:
X represents a phenyl group optionally substituted by one or more groups chosen, independently of one another, from the following atoms or groups: halogen, (C₁-C₆)alkoxy, (C₁-C₆)alkyl or NRaRb, it being possible for the alkyl and alkoxy groups to be optionally substituted by one or more halogen atoms;
R₁ represents a hydrogen atom, a halogen atom, a (C₁-C₆)alkoxy group, a (C₁-C₆)alkyl group, an amino or an NRcRd group; it being possible for the alkyl and alkoxy groups to be optionally substituted by one or more halogen atoms, a hydroxyl group, an amino group or a (C₁-C₆)alkoxy group;
R₂ represents one of the following groups:
• an NRcRd group,
• an -N=CH-NRaRb group,
• a nitro, hydroxyiminoalkyl or alkoxyiminoalkyl group,
• a (C₁-C₆)alkylthio group,
• a (C₁-C₆)alkylsulphinyl group,
• a (C₁-C₆)alkylsulphonyl group,
• an -SO₂-NR₅R₆ group,
• a (((C₁-C₆)alkyl)₃)silylethynyl group;
R₃ represents a hydrogen atom, a (C₁-C₆)alkyl group, a (C₁-C₆)alkoxy group or a halogen atom;
R₄ represents a hydrogen atom, a (C₁-C₄)alkyl group, a (C₁-C₄)alkoxy group or a fluorine atom;
R₅ and R₆, which are identical or different, represent a hydrogen atom or a (C₁-C₆)alkyl group;
Ra and Rb represent, independently of one another, a hydrogen atom or a (C₁-C₆)alkyl group or form, with the nitrogen atom which carries them, a 4- to 7-membered ring;
Rc represents a hydrogen atom and Rd represents a hydrogen atom or a (C₁-C₆)alkyl group, the said alkyl optionally being substituted by a (C₁-C₆)alkoxy group;
in the base form or in the form of an addition salt with an acid.

2. Compounds of formula (I) according to Claim 1, **characterized in that** one of the R₁ or R₂ groups is other than a hydrogen atom.

3. Compounds of formula (I) according to Claim 1 or 2, **characterized in that** R₁ represents a hydrogen atom, a halogen atom or a (C₁-C₆)alkoxy group.

4. Compounds of formula (I) according to any one of the preceding claims, **characterized in that** R₃ and R₄ each represent a hydrogen atom.

5. Compounds of formula (I) according to any one of the preceding claims, **characterized in that** R₂ represents one of the following groups:
• an NRcRd group,
• an -N=CH-NRaRb group,
• a nitro, hydroxyiminoalkyl or alkoxyiminoalkyl group,
• a (C₁-C₆)alkylthio group,
• a (C₁-C₆)alkylsulphinyl group,
• a (C₁-C₆)alkylsulphonyl group,
• an -SO₂-NR₅R₆ group,
• a ((C₁-C₆)alkyl)₃)silylethynyl group;
R₅ and R₆, which are identical or different, represent a hydrogen atom or a (C₁-C₆)alkyl group;
Ra and Rb represent, independently of one another, a hydrogen atom or a (C₁-C₆)alkyl group;
Rc represents a hydrogen atom and Rd represents a hydrogen atom or a (C₁-C₆)alkyl group, said alkyl optionally being substituted by a (C₁-C₆)alkoxy group.

6. Compounds of formula (I) according to any one of the preceding claims, **characterized in that**:
X represents a phenyl group;
R₁ represents a hydrogen atom, a chlorine atom, a methyl group or an ethoxy group;
R₃ and R₄ represent a hydrogen atom;
R₂ represents an NH₂, CH=NOH, NHiPr, nitro, CH=NOMe, NHMe, N=CHNMe₂, NHEt, NHCH₂CH₂OMe, SMe, SOMe, SO₂Me, SO₂NH₂, SO₂NHMe, SO₂NMe₂ or C≡CSiMe₃ group; in the base form or in the form of an addition salt with an acid.

7. Compound according to any one of the preceding claims, **characterized in that** it is chosen from:
6-[(E)-(Hydroxyimino)methyl]-*N*-phenylimidazo[1,2-*a*]-pyridine-2-carboxamide
6-(Isopropylamino)-*N*-phenylimidazo[1,2-*a*]pyridine-2-carboxamide and its hydrochloride (1:1)
6-Nitro-*N*-phenylimidazo[1,2-*a*]pyridine-2-carboxamide
6-Amino-*N*-phenylimidazo[1,2-*a*]pyridine-2-carboxamide
6-Amino-5-chloro-*N*-phenylimidazo[1,2-*a*]pyridine-2-carboxamide
6-Amino-5-ethoxy-*N*-phenylimidaso[1,2-*a*]pyridine-2-carboxamide
6-{[(1E)-(dimethylamino)methylene]amino}-*N*-phenylimidazo[1,2-*a*]pyridine-2-carboxamide
6-[(E)-(methoxyimino)methyl]-*N*-phenylimidazo[1,2-*a*]-pyridine-2-carboxamide
6-(Methylamino)-*N*-phenylimidazo[1,2-*a*]pyridine-2-carboxamide
6-(Ethylamino)-*N*-phenylimidazo[1,2-*a*]pyridine-2-carboxamide
6-(2-Methoxyethylamino)-*N*-phenylimidazo[1,2-*a*]pyridine-2-carboxamide
5-Methyl-6-nitro-*N*-phenylimidazo[1,2-*a*]pyridine-2-carboxamide
6-Amino-5-methyl-*N*-phenylimidazo[1,2-*a*]pyridine-2-carboxamide
6-(Methylthio)-*N*-phenylimidazo[1,2-*a*]pyridine-2-carboxamide and its hydrobromide (1:1)
6-[(RS)-methylsulphinyl]-*N*-phenylimidazo[1,2-*a*]-pyridine-2-carboxamide and its hydrobromide (1:1)
6-(Methylsulphonyl)-*N*-phenylimidazo[1,2-*a*]pyridine-2-carboxamide and its hydrobromide (1:1)
6-(Aminosulphonyl)-*N*-phenylimidazo[1,2-*a*]pyridine-2-carboxamide and its hydrobromide (1:1)
6-[(Methylamino)sulphonyl]-*N*-phenylimidazo[1,2-*a*]-pyridine-2-carboxamide and its hydrobromide (1:1)
6-[(Dimethylamino)sulphonyl]-*N*-phenylimidazo[1,2-*a*]-pyridine-2-carboxamide
6- (Trimethylsilylethynyl) *-N*-phenylimidazo[1,2-*a*]-pyridine-2-carboxamide and its hydrobromide (1:1) and an addition salt of these compounds with a pharmaceutically acceptable acid.

8. Medicament, **characterized in that** it comprises a compound of formula (I) according to any one of Claims 1 to 7 or an addition salt of this compound with a pharmaceutically acceptable acid.

9. Pharmaceutical composition, **characterized in that** it comprises a compound of formula (I) according to any one of Claims 1 to 7 or a pharmaceutically acceptable salt of this compound, and at least one pharmaceutically acceptable excipient,

10. Use of a compound of formula (I) according to any one of Claims 1 to 7 in the preparation of a medicament intended for the treatment and prevention of neurodegenerative diseases.

11. Use of a compound of formula (I) according to any one of Claims 1 to 7 in the preparation of a medicament intended for the treatment and prevention of cerebral traumas and epilepsy.

12. Use of a compound of formula (I) according to any one of Claims 1 to 7 in the preparation of a medicament intended for the treatment and prevention of psychiatric diseases.

13. Use of a compound of formula (I) according to any one of Claims 1 to 7 in the preparation of a medicament intended for the treatment and prevention of inflammatory diseases.

14. Use of a compound of formula (I) according to any one of Claims 1 to 7 in the preparation of a medicament intended for the treatment and prevention of osteoporosis.

15. Use of a compound of formula (I) according to any one of Claims 1 to 7 in the preparation of a medicament intended for the treatment and prevention of cancers.

16. Use of a compound of formula (I) according to any one of Claims 1 to 7 in the preparation of a medicament intended for the treatment and prevention of Parkinson's disease, Alzheimer's disease, tauopathies or multiple sclerosis.

17. Use of a compound of formula (I) according to any one of Claims 1 to 7 in the preparation of a medicament intended for the treatment and prevention of schizophrenia, depression, substance dependence or attention deficit hyperactivity disorders.

18. Intermediate compounds
5-Ethylaminopyridine-2-amine
5- (2-Methoxyethylamino)pyridine-2-amine,

19. Use of the compounds according to Claim 18 in the preparation of the compounds as defined in Claims 1 to 7.
